# EUROPEAN PATENT SPECIFICATION

(11) **EP 2 767 231 B1**
(45) Date of publication and mention of the grant of the patent: **16.09.2020**
(21) Application number: 14154764.6
(22) Date of filing: 12.02.2014
(51) Int. Cl.: A61B 5/0408

(54) **Apparatus and method for processing biosignals**
Vorrichtung und Verfahren zur Verarbeitung von Biosignalen
Dispositif et procédé de traitement de bio-signal

(30) Priority: 13.02.2013 KR 20130015154
(43) Date of publication of application: 20.08.2014
(73) Proprietor: Samsung Electronics Co., Ltd., Gyeonggi-do 443-742 (KR)
(72) Inventor: Ko, Byung Hoon, 446-712 Gyeonggi-do (KR); Kim, Jong Pal, 446-712 Gyeonggi-do (KR); Park, Sang Yun, 446-712 Gyeonggi-do (KR)
(74) Representative: Grünecker Patent- und Rechtsanwälte PartG mbB

(56) References cited:
- EP-A1- 2 394 571
- EP-A1- 2 561 805
- EP-A2- 2 572 635
- US-A- 5 704 365
- US-A1- 2006 074 411
- US-A1- 2011 028 823

## Description

### BACKGROUND

### 1. Field

The following description relates to a bio-electrode configured to measure a biosignal of a user, and an apparatus and method for processing the measured biosignal.

### 2. Description of Related Art

Smart healthcare solutions introduced in recent times may enhance accuracy and convenience of diagnosis by measuring various biosignals of users. Furthermore, healthcare providers may provide improved health-related services by using information from a database of personal health information. For example, smart healthcare solutions may use a bio-electrode configured to measure a biosignal of a user. Examples of these types of measurements include an electrocardiogram (ECG), an electromyography (EMG), an electroencephalogram (EEG), a galvanic skin resistance (GSR), and the like, from the body of a user.

The bio-electrode may be used through being attached to a user's skin, and detect a biopotential representing an electrical phenomena in the user's body. The bio-electrode may be configured as a surface electrode, a needle electrode, a micro electrode, or an alternative type of electrode. In one embodiment, the surface electrode configuration is used. The bio-electrode may include an electrolyte, an adhesive sheet to attach the bio-electrode to a user's skin, a metal electrode, and the like to help measure electrical phenomena in the user's body, and the metal electrode may be electrically and mechanically connected to an apparatus for measuring a biosignal.

EP2394571 discloses a method and apparatuses for measuring a biological signal, in which a biological signal of an examinee is detected via at least one interface that touches skin of the examinee, and a dummy signal is detected via a dummy interface. Noise that is generated by a fluctuation in the electrical characteristics of the at least one interface is removed from the biological signal using the biological signal and the dummy signal.

### Summary

According to the invention there is provided an apparatus for processing a biosignal according to claim 1, including a bio-electrode configured to measure a biosignal of a user, the biosignal comprising a motion artifact, and a motion signal corresponding to the motion artifact, the apparatus being configured to measure an electrical potential difference at different positions. Preferred embodiments are defined in the dependent claims.

Other features and aspects will be apparent from the following detailed description, the drawings, and the claims.

### BRIEF DESCRIPTION OF THE DRAWINGS

FIG. 1 is a diagram illustrating an example of a bio-electrode.
FIG. 2 is a diagram illustrating an example of a configuration of an apparatus for processing a biosignal.
FIG. 3 is a diagram illustrating an example of an apparatus for processing a biosignal.
FIG. 4 is a diagram illustrating an example of a circuit model of a transfer path of a motion based signal.
FIG. 5 is a diagram illustrating an example of a vertical cross-section of a second electrode measuring a motion based signal.
FIG. 6 is a diagram illustrating an example of a circuit model of a transfer path of the motion based signal measured through the second electrode of FIG. 5.
FIG. 7 is a diagram illustrating another example of a configuration of a bio-electrode.
FIG. 8 is a diagram illustrating another example of a configuration of a bio-electrode.
FIG. 9 is a flowchart illustrating an example of a method for processing a biosignal performed by an apparatus for processing a biosignal.

### DETAILED DESCRIPTION

The following detailed description is provided to assist the reader in gaining a comprehensive understanding of the methods, apparatuses, and/or systems described herein. However, various changes, modifications, and equivalents of the systems, apparatuses and/or methods described herein will be apparent to one of ordinary skill in the art. Also, descriptions of functions and constructions that are well known to one of ordinary skill in the art may be omitted for increased clarity and conciseness.

Throughout the drawings and the detailed description, the same reference numerals refer to the same elements. The drawings may not be to scale, and the relative size, proportions, and depiction of elements in the drawings may be exaggerated for clarity, illustration, and convenience.

The features described herein may be embodied in different forms, and are not to be construed as being limited to the examples described herein. Rather, the examples described herein have been provided so that this disclosure will be thorough and complete, and will convey the full scope of the disclosure to one of ordinary skill in the art.

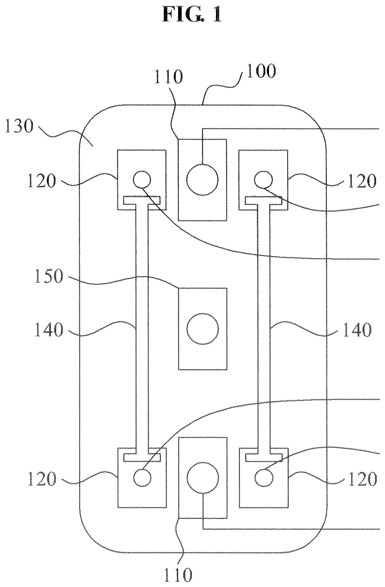

FIG. 1 illustrates an example of a bio-electrode 100.

Referring to FIG. 1, the bio-electrode 100 may include a first electrode 110, a second electrode 120, and a support member 130. The bio-electrode 100 may have a hybrid electrode structure including an electrode configured to measure a biosignal of a user and an electrode configured to measure a motion based signal.

The first electrode 110 may measure the biosignal of the user. In example embodiments, the first electrode 110 measure the biosignal using a technique such as an electrocardiogram (ECG), an electromyography (EMG), an electroencephalogram (EEG), a galvanic skin resistance (GSR), and the like, as a source of an electrical signal. The first electrode 110 may be used for measuring a biopotential difference by being used as a combined electrode as a pair with another first electrode 110. The first electrode 110 may include an adhesive sheet attach it to a user's skin, a metal electrode, an electrolyte that transfers an electrical signal between the metal electrode and the user's skin, and the like.

A motion artifact produced by a motion of the user may be included in the biosignal measured. For example, a relative displacement may occur because an external motion, in the positioning of an interface between a system for measuring a biosignal and the first electrode 110, and the motion artifact may be generated due to an change of an interface when a displacement, such as a displacement resulting from skin of the user being dilated, is transferred to the first electrode 110. The motion artifact may overlap the biosignal due to an electrical property change. In examples, a motion artifact leads to electrical effects such as an impedance or a half cell potential (HCP) on a transfer path of the biosignal. In embodiments where these effects occur, the biosignal becomes distorted due to the motion artifact.

A second electrode 120 may measure a motion based signal that resembles the motion artifact. In embodiments, there may be one second electrode 120 or a plurality of second electrodes 120. For example, there may be one second electrode 120 that is referred to as an initial second electrode, or other, additional second electrodes that are referred to as additional second electrodes.

In embodiments, the motion based signal is used to estimate the motion artifact included in the biosignal. In an example implementation, the second electrode 120 is electrically isolated from the first electrode 110, and may be disposed in an area adjacent to the first electrode 110. The second electrode 120, as with the first electrode 110, includes parts such as an adhesive sheet to attach it to a user's skin, a metal electrode, an electrolyte configured to transfer an electrical signal between the metal electrode and the skin, and the like. The second electrode 120 measures the motion based signal at various points by using a plurality of pairs of the second electrode 120 to monitor the motion based signal, as will be discussed in greater detail hereinafter.

The second electrode 120 may be electrically connected to another second electrode 120 via an electrical connection unit 140 configured to block the biosignal of the user from being transferred. The second electrode 120 may measure a change of an HCP generated in an interface between an electrode and skin, using the electrical connection unit 140. For example, the second electrode 120 may measure an HCP between an electrolyte and a metal electrode, via the electrical connection unit 140.

According to an implementation, an HCP refers to a unique electrode potential of a metal when the metal is disposed to be in parallel with a layer of ions in an electrolyte along a surface of the metal. A double-charge layer may be formed between the metal electrode and the electrolyte or between the electrolyte and the skin. Such a double-charge layer refers to two parallel layers of opposite charge, which attract one another due to the opposing charges.

In one aspect, the HCP is generated due to a change of an ion concentration along the double-charge layer. The HCP generated may be represented using the Nernst Equation. When analyzing the HCP in embodiments, it is possible to ignore a change of an HCP generated between the electrolyte and the skin because a size of the HCP generated between the electrolyte and the skin is considerably smaller than a size of an HCP generated between the metal electrode and the electrolyte.

In an aspect, the second electrode 120 is disposed symmetrically to another second electrode 120 arranged around the first electrode 110. For example, the second electrodes 120 may be disposed symmetrically on a left side and a right side of the first electrode 110, or disposed symmetrically vertically and horizontally with respect to the first electrode 110. Also, the second electrode 120 may include a plurality of sub-electrodes, and the plurality of sub-electrodes may be electrically connected via a conductive material. If the second electrode 120 includes a plurality of sub-electrodes, the sub-electrodes gather information about electrical effects such as HCP or impedance, as discussed above.

According to an aspect, the support member 130 supports the first electrode 110 and the second electrode 120, and firmly fixes the first electrode 110 and the second electrode 120 to the skin of a user. For example, the support member 130 may refer to a pad attached to the skin that maintains the position of the first electrode 110 and the second electrode 120 with respect to the skin of the user.

In one embodiment, the bio-electrode 100 includes a third electrode 150 for a right leg drive circuit. An effect of circuit noise is reduced by applying a bias voltage to the third electrode 150, and a common mode rejection ratio (CMRR) may be enhanced because the bias voltage will increase the ability of the embodiments to discriminate between signals which differ only slightly.

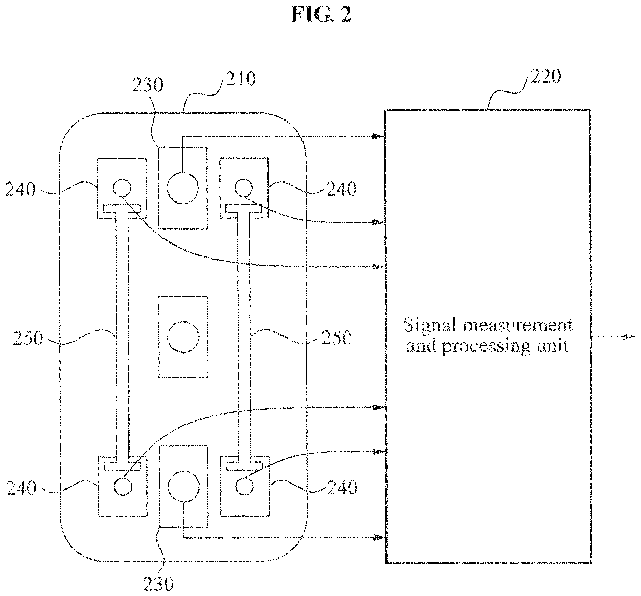

FIG. 2 illustrates an example of a configuration of an apparatus for processing a biosignal.

Referring to FIG. 2, the apparatus for processing the biosignal may include a bio-electrode 210 and a signal measurement and processing unit 220.

In an embodiment, the bio-electrode 210 measures a motion based signal similar to a biosignal of a user and a motion artifact included in the biosignal. The bio-electrode 210 includes a first electrode 230 configured to measure a biosignal of a user and a second electrode 240 configured to measure the motion based signal similar to the motion artifact.

In an example, the bio-electrode 210 has a structure in which a plurality of second electrodes 240, for measuring the motion based signal, are disposed in an area adjacent to the first electrode 230. Optionally, the plurality of second electrodes 240 are electrically connected to other second electrodes 240 via an electrical connection unit 250 configured to block the biosignal of the user from being transferred. Also optionally, the plurality of second electrodes 240 measure an HCP generated on an interface path, using the electrical connection unit 250.

In an embodiment, the plurality of second electrodes 240 are disposed symmetrically to the other second electrodes 240, centered around the first electrode 230, and may include a plurality of sub-electrodes. For additional omitted descriptions of the bio-electrode 210, reference may be made to analogous features described in FIG. 1.

According to one aspect, the signal measurement and processing unit 220 amplifies an amplitude of the biosignal measured by the bio-electrode 210 and an amplitude of the motion based signal, potentially for further processing. For example, the signal measurement and processing unit 220 amplifies an amplitude of a signal measured, using a differential signal amplifier, or a similar component, to a desired amplitude of a signal.

The signal measurement and processing unit 220 may remove a motion artifact from a biosignal, using a motion based signal that models the motion artifact. More particularly, in an embodiment the signal measurement and processing unit 220 estimates the motion artifact included in the biosignal, using the motion based signal. The signal measurement and processing unit 220 then removes or reduce the motion artifact from the biosignal, using the motion artifact estimate that was measured.

For example, the signal measurement and processing unit 220 removes a motion artifact, using an adaptive filtering scheme or an independent component analysis scheme. The independent component analysis scheme may refer to a scheme for separating mutually independent signals from linearly mixed signals using a statistical scheme. Where independent component analysis is used, the signal measurement and processing unit 220 isolates an original biosignal from which a motion artifact is removed from a biosignal mixed with a motion artifact. The isolation occurs by using a statistical scheme, in this example.

In situations where biosignals are of interest, a motion artifact associated with a motion of the user may be measured through being associated with a biosignal when the biosignal is measured by a user using the bio-electrode 210. In an aspect, the motion artifact refers to noise occurring due to an interface change based on the motion of the user. An embodiment may reduce a signal-to-noise ratio (SNR) through simultaneously measuring the motion based signal with the biosignal.

For example, when a system for measuring a biosignal of a user measures an ECG of the user, an error may occur during a detection process of an R-peak, which is a particular event in the context of the ECG. Such potential error may occur due to the aforementioned motion artifact. Such an error may lead to an inaccurate diagnosis with respect to the ECG because the data may not accurately represent the biosignal because of interference from the motion artifact.

The apparatus for processing the biosignal may estimate a motion artifact associated with a biosignal, and remove or decrease an effect of the motion artifact on the measured biosignal. More particularly, in an aspect, the apparatus for processing the biosignal monitors a motion artifact generated due to a change of a dynamic environment or external noise or environmental noise due to outside influences. Based on the results of the monitoring, the apparatus enhances the SNR by removing the motion artifact or the external noise from a biosignal including noise, based on the monitored motion artifact or the monitored external noise.

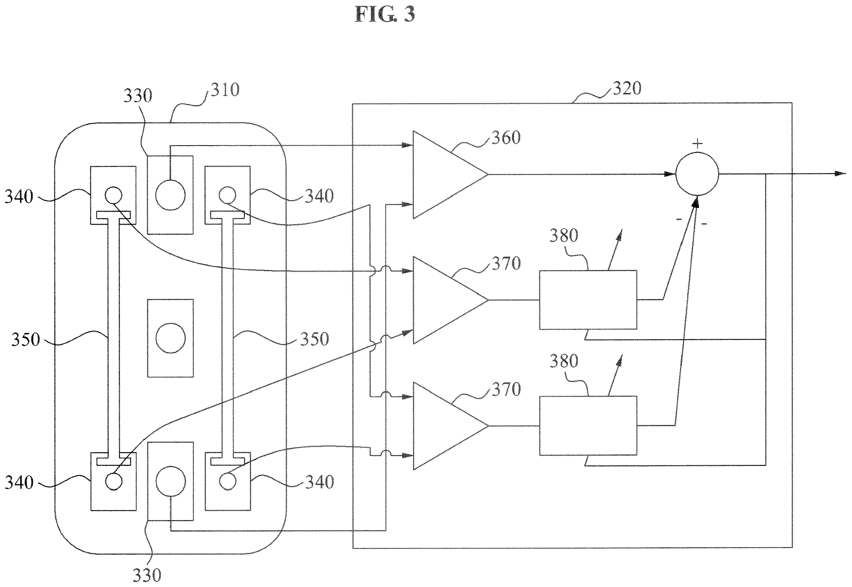

FIG. 3 illustrates an example of an apparatus for processing a biosignal.

Referring to FIG. 3, the apparatus for processing the biosignal may include a bio-electrode 310 and a signal measurement and processing unit 320. The bio-electrode 310 may include a first electrode 330 for measuring a biosignal and a second electrode 340 for measuring a motion based signal similar to a motion artifact included in the biosignal. The second electrode 340 may be connected to another second electrode 340 via an electrical connection unit 350 for blocking a biosignal resulting from an object to be measured from being transferred to the signal measurement and processing unit 320. While not guaranteed to be identical, these features of the apparatus are similar to corresponding features presented in FIGS. 1-2, and further disclosure about these features is presented above.

In an embodiment, the signal measurement and processing unit 320 includes an amplifier 360 for amplifying an amplitude of a biosignal inputted, an amplifier 370 for amplifying an amplitude of a motion based signal, and a filtering unit for removing a motion artifact from a biosignal using a motion based signal. The filtering unit is illustrated as a plurality of adaptive filters 380. The amplifier 360 and the amplifier 370 may amplify signals or differentially amplify a difference of signals measured at two points. In an embodiment, the filtering unit of FIG. 3 performs an adaptive filtering, using a plurality of adaptive filters 380, and additionally adjusts a parameter of a filter, based on an output signal of the signal measurement and processing unit 320.

In an embodiment, the apparatus for processing the biosignal measures a biosignal via the first electrode 330 included in the bio-electrode 310. The biosignal measured may include a motion artifact due to an internal change, such as a motion of a user, and the like, or noise from an external environment. As discussed, both of these types of artifact are detrimental to the quality of the received biosignal.

In an embodiment, the apparatus for processing the biosignal measures a motion based signal similar to the motion artifact, via the second electrode 340 of the bio-electrode 310. The biosignal and the motion based signal measured are amplified through the amplifiers 360 and 370, respectively. The signal measurement and processing unit 320 estimates a form of a motion artifact included in the biosignal from the motion based signal, and remove the motion artifact from the biosignal, based on the form of the estimated motion artifact. After removing the motion artifact, the signal measurement and processing unit 320 output a target signal desired by a user. More particularly, the target signal is a biosignal from which the motion artifact is removed or reduced.

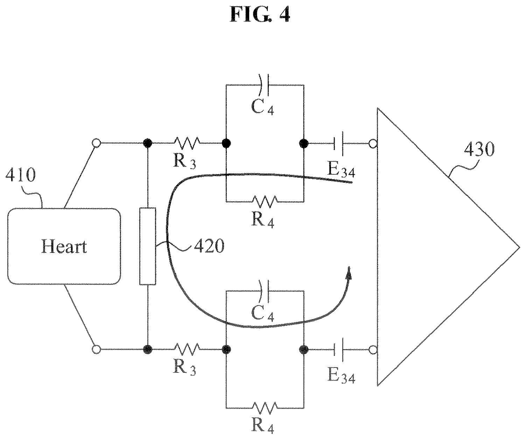

FIG. 4 illustrates an example of a circuit model of a transfer path of a motion based signal.

In an embodiment where a bio-electrode for measuring a potential is attached to skin of a user, an interface model between an electrode and an electrolyte includes resistance components R₃ and R₄ and a capacitor component C₄. The interface model also includes an HCP E₃₄ generated in a double-charge layer in which differing materials are in contact with one another.

An effect of noise due to a change of the resistance components R₃ and R₄ and the capacitor component C₄ may be almost completely disregarded because the amplitudes of the resistance components R₃ and R₄ and the capacitor component C₄ are considerably smaller than the amplitude of an input impedance. However, a change of the HCP E₃₄ generated in the double-charge layer may influence a biosignal measured directly.

FIG. 4 illustrates a closed loop from a signal source, for example, a heart 410, to an amplifier 430 when an apparatus for measuring a biosignal measures an electrical potential difference at differing positions, using second electrodes configured to measure the motion based signal. Both sides on the transfer path of the motion based signal may be connected via an impedance component 420. In an embodiment, the impedance component 420 corresponds to an electrical connection unit electrically connecting two second electrodes measuring the motion based signal.

In the embodiment of FIG. 4, the amplification unit 430 measures an electrical potential difference on an adjacent closed loop by the electrical connection unit, and an electrical potential difference by the signal source 410 may be blocked. The remaining signal provides a way to estimate electrical effects that are not part of the signal.

Accordingly, the apparatus for measuring the biosignal may independently measure noise generated on the transfer path of a signal and a distortion of the signal by the electrical connection unit. The noise generated on the transfer path of the signal and the distortion of the signal may correspond to the motion based signal, which can be used to provide a better version of the biosignal itself.

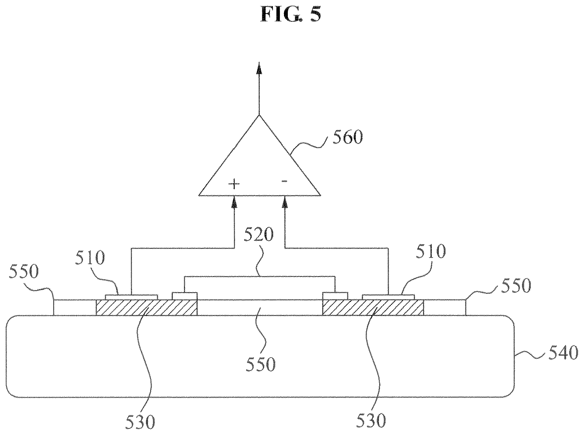

FIG. 5 illustrates an example of a vertical cross-section of a second electrode for measuring a motion based signal.

The second electrode may measure a motion based signal resembling a motion artifact included in a biosignal, through being attached to the skin 540 of a user. For example, the second electrode may include metal electrodes 510 for measuring a motion based signal, a conductive adhesive 530 used as an electrolyte, and an electrical connector 520 for electrically connecting different metal electrodes 510. The second electrode may be supported by a support member 550 that is firmly adheres the second electrode to the skin 540. A signal measured through the metal electrodes 510 may be inputted to an amplifier 560 to be amplified.

The electrical connector 520 may be electrically connected to the metal electrodes 510 through the conductive adhesive 530. An HCP may be generated at a junction of the conductive adhesive 530 and the electrical connector 520 as the electrical connector 520 of the second electrode illustrated in FIG. 5 is disposed on the conductive adhesive 530 as the metal electrodes 510. The electrical connector 520 is not limited in form to the example illustrated in FIG. 5 and be implemented in various forms.

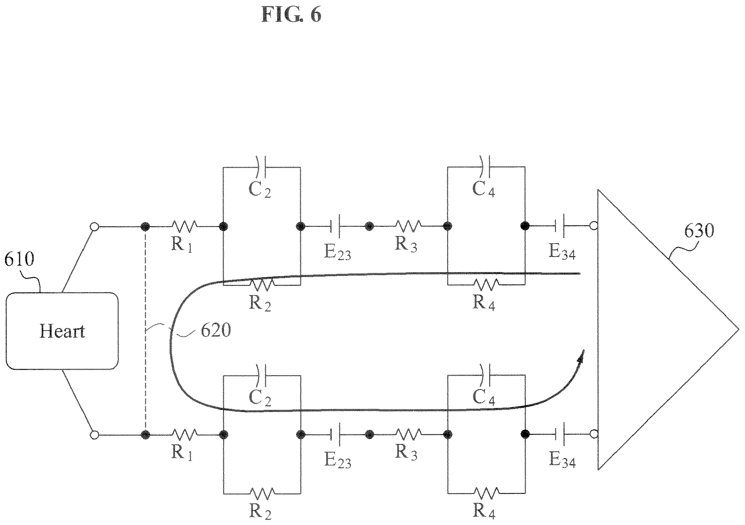

FIG. 6 illustrates an example of a circuit model of a transfer path of the motion based signal measured through the second electrode of FIG. 5.

When an apparatus for measuring a biosignal measures a potential difference at differing positions, it uses second electrodes to measure the motion based signal. In FIG. 6, as shown in FIG. 4, a closed loop from a signal source is illustrated, for example, from a heart 610, to an amplifier 630.

The second electrodes of FIG. 5 may be connected through an electric connection unit, and the electrical connection unit may be attached by a conductive adhesive to a metal electrode. Accordingly, a circuit model representing a transfer path of a motion based signal measured through the second electrodes of FIG. 5 may include an interface model between the electrical connection unit and the conductive adhesive as well as an interface model between a metal electrode and the conductive adhesive.

FIG. 6 illustrates the interface model between the metal electrode and the conductive adhesive and the interface model between the electrical connection unit 620 and the conductive adhesive. An interface model between the electrical connection unit 620 and the conductive adhesive is represented as resistance components R₁ and R₂, a capacitor component C₂, and an HCP E₂₃. The amplifier 630 may measure an electric potential difference on a closed loop by the electrical connection unit 620, and an electric potential difference by the signal source 610 may be removed to improve the quality of the biosignal.

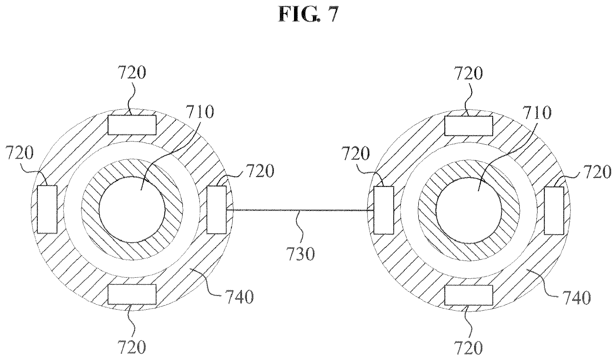

FIG. 7 illustrates another example of a configuration of a bio-electrode.

In one example a second electrode includes a plurality of sub-electrodes 720. The plurality of sub-electrodes 720 may be separated from a first electrode 710, and may be electrically connected through a conductive material 740. The plurality of sub-electrodes 720 may be disposed at predetermined intervals apart from one another centered around the first electrode 710, or disposed in a form of concentric circle. At least one of the plurality of sub-electrodes 720 may be electrically connected to at least one of the plurality of sub-electrodes 720 included in another second electrode, through an electrical connection unit 730.

The plurality of sub-electrodes 720 may be disposed symmetrically, centered around the first electrode 710. A similarity or a correlation between a motion artifact included in a biosignal measured by the first electrode 710 and the motion based signal may be enhanced in an embodiment where the plurality of sub-electrodes 720 for measuring a motion based signal are disposed symmetrically with respect to the first electrode 710.

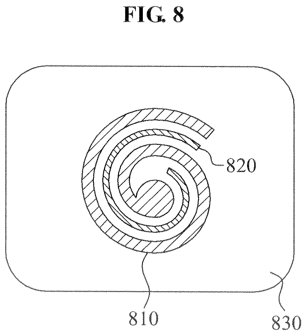

FIG. 8 illustrates another example of a configuration of a bio-electrode.

A second electrode 820 may be provided in a spiral form, while still being separated from a first electrode 810 electrically. An accuracy of a motion based signal with respect to a motion artifact may be enhanced through the first electrode 810 having a spiral form and the second electrode 820 having a spiral form, where the second electrode 820 is formed as a spiral that is disposed to be adjacent to the first electrode 810.

Anisotropic hydrogel may be used as an electrolyte 830 as part of an interface between the first electrode 810 and a user's skin and an interface between the second electrode 820 and a user's skin. Anisotropic hydrogel may have an electrical property in which the anisotropic hydrogel has a conductivity only in a vertical direction, and does not conduct electricity in a direction other than the vertical direction. Accordingly, the first electrode 810 and the second electrode 820 may be electrically separated through anisotropic hydrogel by constraining the direction in which electricity is conducted.

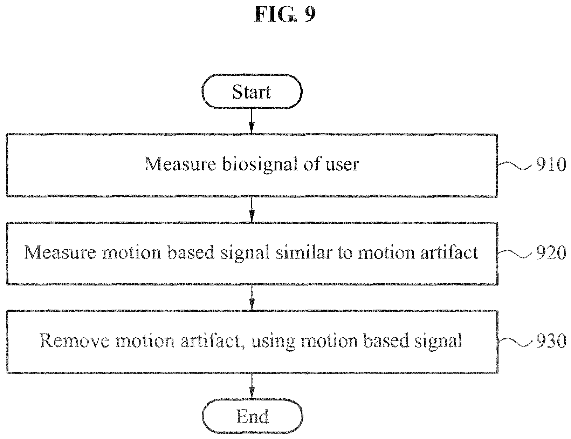

FIG. 9 illustrates an example of a method for processing a biosignal performed by an apparatus for processing a biosignal.

In 910, the apparatus for processing the biosignal may measure a biosignal of a user. The apparatus for processing the biosignal may measure a biosignal through a first electrode included in a bio-electrode, and amplify the biosignal measured through an amplifier. Examples of measuring a biosignal have been discussed, above.

In 920, the apparatus for processing the biosignal may measure a motion based signal similar to a motion artifact included in the biosignal. In such a measurement, the motion based signal provides an estimate of a motion artifact included in the biosignal. The apparatus for processing the biosignal may measure a motion based signal through a second electrode, and amplify the motion based signal measured through an amplifier.

The second electrode may be disposed in an area adjacent to the first electrode, and may be disposed symmetrically to another second electrode, centered around the first electrode. The second electrode may be electrically connected to the other second electrode through an electrical connection unit for blocking a biosignal of a user from being transferred when estimating the motion artifact. Also, the second electrode may be configured to include plurality of sub-electrodes, and at least one of the plurality of sub-electrodes may be electrically connected to at least one of a plurality of sub-electrodes included in the other second electrode, via the electrical connection unit.

The sequence of operations are not limited to the example described in the foregoing, in particular, 920 may be performed prior to 910 or performed simultaneously with 910.

In 930, the apparatus for processing the biosignal may remove or reduce a motion artifact from a biosignal, using a motion based signal. More particularly, the apparatus for processing the biosignal may estimate the motion artifact included in the biosignal, using the motion based signal, and remove the motion artifact from the biosignal, using the estimated motion artifact. The apparatus for processing the biosignal may remove the motion artifact, using an adaptive filtering scheme or an independent component analysis scheme. The apparatus for processing the biosignal may separate an original biosignal from which a motion artifact is removed, from a biosignal mixed with a motion artifact by a statistical scheme when the independent component analysis scheme is used.

The examples of a bio-electrode configured to measure a biosignal of a user, and an apparatus and method for processing the measured biosignal may improve the quality of biosignal information to aid in health care and medical diagnostics and treatment of the user.

A hardware component may be, for example, a physical device that physically performs one or more operations, but is not limited thereto. Examples of hardware components include microphones, amplifiers, low-pass filters, high-pass filters, band-pass filters, analog-to-digital converters, digital-to-analog converters, and processing devices.

A software component may be implemented, for example, by a processing device controlled by software or instructions to perform one or more operations, but is not limited thereto. A computer, controller, or other control device may cause the processing device to run the software or execute the instructions. One software component may be implemented by one processing device, or two or more software components may be implemented by one processing device, or one software component may be implemented by two or more processing devices, or two or more software components may be implemented by two or more processing devices.

A processing device may be implemented using one or more general-purpose or special-purpose computers, such as, for example, a processor, a controller and an arithmetic logic unit, a digital signal processor, a microcomputer, a field-programmable array, a programmable logic unit, a microprocessor, or any other device capable of running software or executing instructions. The processing device may run an operating system (OS), and may run one or more software applications that operate under the OS. The processing device may access, store, manipulate, process, and create data when running the software or executing the instructions. For simplicity, the singular term "processing device" may be used in the description, but one of ordinary skill in the art will appreciate that a processing device may include multiple processing elements and multiple types of processing elements. For example, a processing device may include one or more processors, or one or more processors and one or more controllers. In addition, different processing configurations are possible, such as parallel processors or multi-core processors.

Software or instructions for controlling a processing device to implement a software component may include a computer program, a piece of code, an instruction, or some combination thereof, for independently or collectively instructing or configuring the processing device to perform one or more desired operations. The software or instructions may include machine code that may be directly executed by the processing device, such as machine code produced by a compiler, and/or higher-level code that may be executed by the processing device using an interpreter. The software or instructions and any associated data, data files, and data structures may be embodied permanently or temporarily in any type of machine, component, physical or virtual equipment, computer storage medium or device, or a propagated signal wave capable of providing instructions or data to or being interpreted by the processing device. The software or instructions and any associated data, data files, and data structures also may be distributed over network-coupled computer systems so that the software or instructions and any associated data, data files, and data structures are stored and executed in a distributed fashion.

As a non-exhaustive illustration only, a terminal/device/unit described herein may be a mobile device, such as a cellular phone, a personal digital assistant (PDA), a digital camera, a portable game console, an MP3 player, a portable/personal multimedia player (PMP), a handheld e-book, a portable laptop PC, a global positioning system (GPS) navigation device, a tablet, a sensor, or a stationary device, such as a desktop PC, a high-definition television (HDTV), a DVD player, a Blue-ray player, a set-top box, a home appliance, or any other device known to one of ordinary skill in the art that is capable of wireless communication and/or network communication.

The processes, functions, methods and/or software described above including a method for processing a biosignal may be recorded, stored, or fixed in one or more non-transitory computer-readable storage media that includes program instructions to be implemented by a computer to cause a processor to execute or perform the program instructions. The media may also include, alone or in combination with the program instructions, data files, data structures, and the like. The media and program instructions may be those specially designed and constructed, or they may be of the kind well-known and available to those having skill in the computer software arts. Examples of non-transitory computer-readable media include magnetic media such as hard disks, floppy disks, and magnetic tape; optical media such as CD ROM discs and DVDs; magneto-optical media such as optical discs; and hardware devices that are specially configured to store and perform program instructions, such as read-only memory (ROM), random access memory (RAM), flash memory, and the like. Examples of program instructions include both machine code, such as produced by a compiler, and files containing higher level code that may be executed by the computer using an interpreter. The described hardware devices may be configured to act as one or more software modules in order to perform the operations and methods described above, or vice versa. In addition, a non-transitory computer-readable storage medium may be distributed among computer systems connected through a network and non-transitory computer-readable codes or program instructions may be stored and executed in a decentralized manner.

While this disclosure includes specific examples, it will be apparent to one of ordinary skill in the art that various changes in form and details may be made in these examples without departing from the spirit and scope of the claims and their equivalents. The examples described herein are to be considered in a descriptive sense only, and not for purposes of limitation. Descriptions of features or aspects in each example are to be considered as being applicable to similar features or aspects in other examples. Suitable results may be achieved if the described techniques are performed in a different order, and/or if components in a described system, architecture, device, or circuit are combined in a different manner and/or replaced or supplemented by other components or their equivalents. Therefore, the scope of the disclosure is defined not by the detailed description, but by the claims and their equivalents, and all variations within the scope of the claims and their equivalents are to be construed as being included in the disclosure.

## Claims

1. An apparatus for processing a biosignal, comprising:
a bio-electrode (100, 210, 310), the bio-electrode being configured to measure a biosignal of a user, the biosignal comprising a motion artifact, and to measure a motion signal corresponding to the motion artifact, and
a signal measurement and processing unit (220, 320) configured to remove the motion artifact from the biosignal using the motion signal;
wherein the bio-electrode comprises:
a first electrode (110, 230, 330) configured to measure the biosignal;
at least two second electrodes (120, 240, 340) configured to measure the motion signal corresponding to the motion artifact, the at least two second electrodes being disposed symmetrically to each other and centered around the first electrode; and
an electrical connection unit (140, 250, 350, 420, 620) configured to electrically connect one of the at least two second electrodes to another one of the at least two second electrodes;
**characterized in that**
the apparatus is configured to measure an electrical potential difference at different positions using the at least two second electrodes; and
the electrical connection unit is further configured to block the biosignal of the user from being measured or transferred when measuring the motion signal, by connecting both sides of a transfer path of the motion signal through the one of the at least two second electrodes and the other one of the at least two second electrodes, respectively, to the signal measurement and processing unit (220, 320).

2. The apparatus of claim 1, wherein each of the at least two second electrodes is configured to measure a half cell potential, HCP, occurring on an interface path, using the electrical connection unit.

3. The apparatus of claim 1 or 2, wherein each of the at least two second electrodes comprises sub-electrodes electrically connected to each other via a conductive material.

4. The apparatus of claim 3, wherein at least one of the sub-electrodes of the one of the at least two second electrodes is configured to be electrically connected to at least one of the sub-electrodes included in the other one of the at least two second electrodes.

5. The apparatus of any one of claims 1 to 4, wherein the bio-electrode further comprises:
a support member (130) to support the first electrode and the at least two second electrodes in proximity to the user.

6. The apparatus according to any one of claims 1 to 5;
wherein the signal measurement and processing unit (220, 320) is configured to remove the motion artifact from the biosignal, using the motion signal by an adaptive filtering scheme or an independent component analysis scheme.

7. A method for processing a biosignal using the apparatus according to any one of claims 1 to 6, the method comprising:
measuring a biosignal of a user;
measuring a motion signal that provides an estimate of a motion artifact included in the biosignal; and
removing the motion artifact from the biosignal, using the motion signal by an adaptive filtering scheme or an independent component analysis scheme.

## Patentansprüche

1. Vorrichtung zum Verarbeiten eines Biosignals, umfassend:
eine Bioelektrode (100, 210, 310), wobei die Bioelektrode dazu eingerichtet ist, ein Biosignal eines Benutzers zu messen, wobei das Biosignal ein Bewegungsartefakt umfasst, und ein dem Bewegungsartefakt entsprechendes Bewegungssignal zu messen, und
eine Signalmess- und -verarbeitungseinheit (220, 320), die dazu eingerichtet ist, das Bewegungsartefakt unter Verwendung des Bewegungssignals aus dem Biosignal zu entfernen;
wobei die Bioelektrode umfasst:
eine erste Elektrode (110, 230, 330), die zum Messen des Biosignals eingerichtet ist;
wenigstens zwei zweite Elektroden (120, 240, 340), die dazu eingerichtet sind, das dem Bewegungsartefakt entsprechende Bewegungssignal zu messen, wobei die wenigstens zwei zweiten Elektroden symmetrisch zueinander angeordnet und um die erste Elektrode zentriert sind; und
eine elektrische Verbindungseinheit (140, 250, 350, 420, 620) die dazu eingerichtet ist, eine der wenigstens zwei zweiten Elektroden elektrisch mit einer anderen der wenigstens zwei zweiten Elektroden zu verbinden;
**dadurch gekennzeichnet, dass**
die Vorrichtung dazu eingerichtet ist, eine elektrische Potentialdifferenz an verschiedenen Positionen unter Verwendung der wenigstens zwei zweiten Elektroden zu messen; und
die elektrische Verbindungseinheit weiterhin dazu eingerichtet ist zu verhindern, dass das Biosignal des Benutzers gemessen oder übertragen wird, wenn das Bewegungssignal gemessen wird, indem beide Seiten eines Übertragungsweges des Bewegungssignals durch die eine der wenigstens zwei zweiten Elektroden bzw. die andere der wenigstens zwei zweiten Elektroden zur Signalmess- und -verarbeitungseinheit (220, 320) verbunden werden.

2. Vorrichtung nach Anspruch 1, bei der jede der wenigstens zwei zweiten Elektroden dazu eingerichtet ist, ein Halbzellenpotential HCP, das auf einem Schnittstellenpfad auftritt, unter Verwendung der elektrischen Verbindungseinheit zu messen.

3. Vorrichtung nach Anspruch 1 oder 2, bei der jede der wenigstens zwei zweiten Elektroden Nebenelektroden umfasst, die über ein leitendes Material elektrisch miteinander verbunden sind.

4. Vorrichtung nach Anspruch 3, bei der wenigstens eine der Unterelektroden der einen der wenigstens zwei zweiten Elektroden so konfiguriert ist, dass sie elektrisch mit wenigstens einer der Unterelektroden verbunden ist, die in der anderen der wenigstens zwei zweiten Elektroden enthalten sind.

5. Vorrichtung nach einem der Ansprüche 1 bis 4, bei der die Bioelektrode weiterhin umfasst:
ein Halteelement (130) zum Halten der ersten Elektrode und der wenigstens zwei zweiten Elektroden in der Nähe des Benutzers.

6. Vorrichtung nach einem der Ansprüche 1 bis 5;
bei der die Signalmess- und -verarbeitungseinheit (220, 320) dazu eingerichtet ist, das Bewegungsartefakt aus dem Biosignal zu entfernen, wobei das Bewegungssignal durch ein adaptives Filterschema oder ein unabhängiges Komponentenanalyseschema verwendet wird.

7. Verfahren zur Verarbeitung eines Biosignals unter Verwendung der Vorrichtung nach einem der Ansprüche 1 bis 6, wobei das Verfahren umfasst:
Messen eines Biosignals eines Benutzers;
Messen eines Bewegungssignals, das eine Schätzung eines in dem Biosignal enthaltenen Bewegungsartefakts liefert; und
Entfernen des Bewegungsartefakts aus dem Biosignal unter Verwendung des Bewegungssignals durch ein adaptives Filterschema oder ein unabhängiges Komponentenanalyseschema.

## Revendications

1. Appareil pour le traitement d'un bio-signal, comprenant:
une bio-électrode (100, 210, 310), la bio-électrode étant configurée pour mesurer un bio-signal d'un utilisateur, le bio-signal comprenant un artefact de mouvement, et pour mesurer un signal de mouvement correspondant à l'artefact de mouvement, et
une unité de mesure et de traitement du signal (220, 320) configurée pour éliminer l'artefact de mouvement du bio-signal en utilisant le signal de mouvement;
dans lequel la bio-électrode comprend:
une première électrode (110, 230, 330) configurée pour mesurer le biosignal;
au moins deux secondes électrodes (120, 240, 340) configurées pour mesurer le signal de mouvement correspondant à l'artefact de mouvement, lesdites au moins deux secondes électrodes étant disposées symétriquement l'une par rapport à l'autre et centrées autour de la première électrode; et
une unité de connexion électrique (140, 250, 350, 420, 620) configurée pour connecter électriquement une desdites au moins deux secondes électrodes à une autre des au moins deux secondes électrodes;
**caractérisé en ce que**
l'appareil est configuré pour mesurer une différence de potentiel électrique à différentes positions en utilisant les deux secondes électrodes au moins; et
l'unité de connexion électrique est en outre configurée pour empêcher que le bio-signal de l'utilisateur soit mesuré ou transféré lors de la mesure du signal de mouvement, en connectant les deux côtés d'un chemin de transfert du signal de mouvement par l'une desdites au moins deux secondes électrodes et l'autre desdites au moins deux secondes électrodes, respectivement, à l'unité de mesure et de traitement du signal (220, 320).

2. Appareil selon la revendication 1, dans lequel chacune desdites au moins deux secondes électrodes est configurée pour mesurer un demi potentiel de cellule, HCP, se produisant sur un chemin d'interface, en utilisant l'unité de connexion électrique.

3. Appareil selon les revendications 1 ou 2, dans lequel chacune desdites deux secondes électrodes au moins comprend des sous-électrodes reliées électriquement entre elles par un matériau conducteur.

4. Appareil selon la revendication 3, dans lequel au moins une desdites sous-électrodes de l'une des au moins deux secondes électrodes est configurée pour être connectée électriquement à au moins une desdites sous-électrodes incluses dans l'autre des au moins deux secondes électrodes.

5. Appareil selon l'une des revendications 1 à 4, dans lequel la bio-électrode comprend en outre:
un élément de support (130) pour soutenir la première électrode et les au moins deux secondes électrodes à proximité de l'utilisateur.

6. Appareil selon l'une des revendications 1 à 5;
dans lequel l'unité de mesure et de traitement du signal (220, 320) est configurée pour éliminer l'artefact de mouvement du bio-signal, en utilisant le signal de mouvement par un schéma de filtrage adaptatif ou un schéma d'analyse de composants indépendants.

7. Procédé de traitement d'un bio-signal utilisant l'appareil selon l'une quelconque des revendications 1 à 6, le procédé comprenant:
la mesure du bio-signal d'un utilisateur;
la mesure d'un signal de mouvement qui fournit une estimation d'un artefact de mouvement inclus dans le bio-signal
l'élimination de l'artefact de mouvement du bio-signal, en utilisant le signal de mouvement par un schéma de filtrage adaptatif ou un schéma d'analyse des composantes indépendantes.
